(19) Europäisches Patentamt / European Patent Office / Office européen des brevets

(11) **EP 2 194 956 B1**

(12) **EUROPÄISCHE PATENTSCHRIFT**

(45) Veröffentlichungstag und Bekanntmachung des Hinweises auf die Patenterteilung:
**12.04.2017 Patentblatt 2017/15**

(51) Int Cl.:
*A61K 8/06* *(2006.01)*     *A61K 8/37* *(2006.01)*
*A61K 8/60* *(2006.01)*     *A61K 8/73* *(2006.01)*
*A61K 8/81* *(2006.01)*     *A61Q 5/02* *(2006.01)*
*A61Q 5/12* *(2006.01)*

(21) Anmeldenummer: **08773440.6**

(22) Anmeldetag: **14.06.2008**

(86) Internationale Anmeldenummer:
**PCT/EP2008/004803**

(87) Internationale Veröffentlichungsnummer:
**WO 2008/155075 (24.12.2008 Gazette 2008/52)**

(54) **SHAMPOO-ZUSAMMENSETZUNG MIT VERBESSERTER PFLEGELEISTUNG**

SHAMPOO COMPOSITION HAVING IMPROVED CARE PROPERTIES

COMPOSITION DE SHAMPOOING À PERFORMANCES AMÉLIORÉES EN TERMES DE SOINS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: 19.06.2007 EP 07011967
27.09.2007 DE 102007046575
03.04.2008 DE 102008017032
03.04.2008 DE 102008017034
07.05.2008 DE 102008022433
21.05.2008 DE 102008024570

(43) Veröffentlichungstag der Anmeldung:
**16.06.2010 Patentblatt 2010/24**

(73) Patentinhaber: **Cognis IP Management GmbH**
**40589 Dusseldorf (DE)**

(72) Erfinder:
• **HLOUCHA, Matthias**
**50677 Köln (DE)**
• **HAAKE, Hans-Martin**
**40699 Erkrath (DE)**
• **KÜSTERS, Esther**
**40625 Düsseldorf (DE)**
• **MENZER, Jasmin**
**40789 Monheim (DE)**
• **EISFELD, Wolf**
**40764 Langenfeld (DE)**
• **SEIPEL, Werner**
**D-40723 Hilden (DE)**
• **HENSEN, Hermann**
**D-42781 Haan (DE)**
• **GEHM, Esther, Ricarda**
**67655 Kaiserslautern (DE)**

(74) Vertreter: **BASF IP Association**
**BASF SE**
**G-FLP-C006**
**67056 Ludwigshafen (DE)**

(56) Entgegenhaltungen:
**WO-A1-99/53889**      **DE-A1- 10 241 296**
**DE-A1-102004 030 885**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung befindet sich auf dem Gebiet der kosmetischen Mittel zum Konditionieren von Haut und Haaren, die Mikroemulsionen und Polymere enthalten.

Nach der Wäsche fühlen sich Haut und Haare häufig rauh und spröde an, insbesondere wenn sie schon durch Umwelteinflüsse vorgeschädigt sind. Haare können überdies noch durch Färben oder Dauerwellen geschädigt sein und zeichnen sich dann nach der Haarwäsche oft durch einen trockenen strohigen Griff aus.

Daher werden in Shampoo-Zusammensetzungen häufig Konditionierer eingesetzt, die diesen Nachteilen entgegen wirken sollen. Häufig findet man daher Shampoo-Zusammensetzungen, die als Konditionierer Silikone enthalten. Diese können jedoch irreversibel auf die Haare aufziehen und verursachen so ihrerseits negative Auswirkungen auf den Griff, im schlimmsten Fall sogar zu Problemen beim Färben und Dauerwellen von Haaren.

Weiterhin kommen als Konditionierungsmittel in diesen kosmetischen Zubereitungen Öle und Wachse in Frage. Diese sind in ihrer Wirkung jedoch bei weitem nicht so ausgeprägt wie die o.g. Silikone. Ausserdem sind durch Verwendung dieser Konditionierer nur noch trübe Formulierungen möglich bzw. können diese Öle und Wachse sowieso nur in geringen Mengen in den Zubereitungen stabilisiert werden.

Durch den Einsatz von alkoxylierten Tensiden, meist Alkylethersulfaten, können bei der Anwendung der kosmetischen Mittel Hautirritationen auftreten und ausserdem mehren sich die Rufe nach "grünen Kosmetika", die frei von alkoxylierten Verbindungen sind. Für diese Einschränkungen bezüglich der Tenside gibt es bisher keine befriedigende Lösung, um Shampoos mit guter Konditionierleistung bereitzustellen. Transparente konditionierende Haarshampoos sind aus DE 10 2004 030 885 bekannt.

Die Aufgabe der vorliegenden Erfindung hat somit darin bestanden, kosmetische Mittel bereit zu stellen, deren Konditionierungsleistung der von silikonhaltigen Zubereitungen entspricht oder bestenfalls diese sogar noch übertrifft und die bei Vermeidung von alkoxylierten Inhaltsstoffen mindestens gleich gute Schaumeigenschaften aufweisen wie Zusammensetzungen auf Basis von alkoxylierten Inhaltsstoffen.

Überraschenderweise wurde gefunden, dass eine kosmetische Zubereitung gemäß Anspruch 1 die oben genannte Aufgabe löst.

Durch Einarbeitung einer derartigen Mikroemulsion als Komponente (b) der erfindungsgemäßen Zubereitungen gelingt die transparente und stabile Einarbeitung größerer Mengen von Ölkörpem, die dann synergistisch mit den kationischen Polymeren der Komponente (c) in der Zusammensetzung stabilisiert durch die Tenside der Komponente (a) die hervorragenden konditionierenden Eigenschaften der Zubereitung bewirken.

Tenside

Als Komponente a) können nicht-alkoxylierte anionische, zwitterionische oder amphotere Tenside enthalten sein. Typische Beispiele für **anionische Tenside** sind Seifen, Alkylbenzolsulfonate, Alkansulfonate, Olefinsulfonate, $\alpha$-Methylestersulfonate, Sulfofettsäuren, Alkylsulfate, Monoglyceridsulfate, Fettsäureamidsulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Alkyloligoglucosidcarboxylate, Proteinfettsäurekondensate (insbesondere pflanzliche Produkte auf Weizenbasis) und Alkylphosphate. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bevorzugt ist Alkylsulfat und besonders bevorzugt eine Kombination von Alkylsulfat und Cocamidopropylbetain, ganz besonders bevorzugt eine Kombination von Laurylsulfat und Cocamidopropylbetain.

Mikroemulsion

Die Mikroemulsionen der Komponente (b) besitzen eine mittlere Teilchengröße von weniger als 1 $\mu$m.

Hergestellt werden diese Emulsionen indem man zunächst in einem ersten Schritt eine Mikroemulsion herstellt, enthaltend mindestens 10 - 20 Gew.-% eines Alkyl(oligo)glycosids der allgemeinen Formel $R^1O\text{-}[G]_p$ in der $R^1$ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht und 4 - 10 Gew.-% eines Monoesters von Glycerin mit einer gesättigten oder ungesättigten Fettsäure der Kettenlänge C12-C22 und 5 - 30 Gew.-% eines Ölkörpers und den Rest auf 100 Gew.-% Wasser.

Unter Mikroemulsionen werden zunächst alle makroskopisch homogenen, optisch transparenten, niedrigviskosen und insbesondere thermodynamisch stabilen Mischungen aus zwei miteinander nicht mischbaren Flüssigkeiten und mindestens einem nichtionischen oder einem ionischen Tensid verstanden. Die mittleren Teilchengrößen der Mikroemulsionen liegen üblicherweise unter 100 nm, sie weisen eine hohe Transparenz auf und sind beim Zentrifugieren bei 2000 UPM für mindestens 30 Minuten gegenüber einer sichtbaren Phasenseparation stabil.

Die Herstellung der Mikroemulsionen erfolgt vorzugsweise einfach durch Vermischen der Ölphase mit den weiteren öllöslichen Inhaltsstoffen, Erwärmen der Ölphase über den Schmelzpunkt aller Bestandteile und anschließender Zugabe der wässrigen tensidhaltigen Phase. Die thermodynamisch stabile Mikroemulsion bildet sich dann spontan, ggf. muss noch etwas gerührt werden.

Die Mikroemulsion enthält als zwingende Bestandteile ein Zuckertensid, und zwar ein Alkyl(oligo)glycosid (im Folgenden auch als "APG" bezeichnet). Alkyl- und/oder Alkenyloligoglycoside im Sinne der vorliegenden Lehre folgen dabei der Formel $R^1O-[G]_p$ in der $R^1$ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,5 liegt. APGs sind in den Mikroemulsionen gemäß der vorliegenden Erfindung in Mengen zwischen 10 und 20 Gew.-%, jeweils bezogen auf die Gesamtmenge der Mikroemulsion enthalten. Besonders bevorzugt sind dabei Mengen im Bereich von 14 bis 19 Gew.-%.

Weiterhin sind Monoester aus Fettsäuren der Kettenlänge C12-C22 mit Glycerin in den Emulsionen enthalten. Dabei sind insbesondere Monoester von Glycerin mit ungesättigten linearen Fettsäuren geeignet. Besonders bevorzugt im Sinne der Erfindung ist Glycerinmonooleat. Diese Glycerinester sind in den Mikroemulsionen in Mengen von 4 bis 10 Gew.-%, vorzugsweise 5 bis 9 Gew.-% - jeweils bezogen auf das Gesamtgewicht der Mikroemulsion - enthalten.

Schließlich enthalten die Mikroemulsionen noch einen Ölkörper, also eine nichtwasserlösliche organische Phase in Mengen von 5 bis 30 Gew.-%. Dabei sind besonders bevorzugte Ölphasen ausgewählt aus der Gruppe von Guerbetalkoholen auf Basis von Fettalkoholen mit 6 bis 18 C-Atomen, Estern linearer $C_6$-$C_{22}$-Fettsäuren mit linearen oder verzweigten $C_6$-$C_{22}$-Fettalkoholen bzw. Estern von verzweigten $C_6$-$C_{13}$-Carbonsäuren mit linearen oder verzweigten $C_6$-$C_{22}$-Fettalkoholen, Ester von linearen $C_6$-$C_{22}$-Fettsäuren mit verzweigten Alkoholen, Estern von $C_6$-$C_{22}$-Fettalkoholen und/oder Guerbetalkoholen mit aromatischen Carbonsäuren, Triglyceriden auf Basis $C_6$-$C_{10}$-Fettsäuren, flüssigen Mono-/Di-/Triglyceridmischungen auf Basis von $C_6$-$C_{18}$-Fettsäuren, Estern von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Öle, verzweigten primäre Alkoholen, substituierten Cyclohexanen, linearen und verzweigten $C_6$-$C_{22}$-Fettalkoholcarbonaten, Guerbetcarbonaten auf Basis von Fettalkoholen mit 6 bis 18, vorzugsweise 8 bis 10 C Atomen, Estern der Benzoesäure mit linearen und/oder verzweigten $C_6$-$C_{22}$-Alkoholen, linearen oder verzweigten, symmetrischen oder unsymmetrischen Dialkylethern mit 6 bis 22 Kohlenstoffatomen pro Alkylgruppe, aliphatischen bzw. naphthenischen Kohlenwasserstoffe, Dialkylcyclohexanen und/oder Silikonölen. Als Ölkomponente können jedoch auch feste Fette und/oder Wachse. Diese können auch in Mischung mit den im vorherigen Abschnitt genannten Ölen vorliegen. Typische Beispiele für Fette sind Glyceride, d.h. feste oder flüssige pflanzliche oder tierische Produkte, die im Wesentlichen aus gemischten Glycerinestern höherer Fettsäuren bestehen. Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Tocopherole und ätherische Öle eignen sich ebenfalls als Ölkomponente.

Als Kohlenwasserstoffe werden organische Verbindungen bezeichnet, die nur aus Kohlenstoff und Wasserstoff bestehen. Sie umfassen sowohl cyclische als auch acyclische (=aliphatische) Verbindungen. Sie umfassen sowohl gesättigte wie einfach oder mehrfach ungesättigte Verbindungen. Die Kohlenwasserstoffe können linear oder verzweigt sein. Je nach Anzahl der Kohlenstoffatome im Kohlenwasserstoff kann man die Kohlenwasserstoffe einteilen in ungradzahlige Kohlenwasserstoffe (wie beispielsweise Nonan, Undecan, Tridecan) oder geradzahlige Kohlenwasserstoffe (wie beispielsweise Octan, Dodecan, Tetradecan). Je nach Art der Verweigung kann man die Kohlenwasserstoffe einteilen in lineare (= unverzweigte) oder verzweigte Kohlenwasserstoffe. Gesättigte, aliphatische Kohlenwasserstoffe werden auch als Paraffine bezeichnet.

Als "Kohlenwasserstoff Gemisch" werden Mischungen von Kohlenwasserstoffen verstanden, die bis zu 10 Gew.-% Substanzen enthalten, die nicht zu den Kohlenwasserstoffen zählen. Die Gew.-% Angaben der linearen C11 und linearen C 13 Kohlenwasserstoffe beziehen sich jeweils auf die Summe der im Gemisch vorhandenen Kohlenwasserstoffe. Die bis zu 10 Gew.-% vorhandenen Nicht-Kohlenwasserstoffe werden für diese Berechnung nicht berücksichtigt.

## EP 2 194 956 B1

Bei den Substanzen, die nicht zu den Kohlenwasserstoffen zählen und die bis zu 10 Gew.-%, insbesondere bis zu 8 Gew.-%, vorzugsweise bis zu 5 Gew.-% im Kohlenwasserstoff-Gemisch enthalten sein können, handelt sich beispielsweise um Fettalkohole, die als nicht umgesetzte Edukte im Kohlenwasserstoff Gemisch verbleiben.

Der Begriff "CX-Kohlenwasserstoff" umfasst Kohlenwasserstoffe mit einer C-Zahl von X, so umfasst beispielsweise der Begriff C11-Kohlenwasserstoff alle Kohlenwasserstoffe mit einer C-Zahl von 11.

Bevorzugt werden Kohlenwasserstoff-Gemische, wobei das Gemisch enthält

    (a) 50 bis 90 Gew.-% lineare C-11 Kohlenwasserstoffe, vorzugsweise n-Undecan
    (b) 10 bis 50 Gew.-% lineare C13 Kohlenwasserstoffe, vorzugsweise n-Tridecan

bezogen auf die Summe der Kohlenwasserstoffe.

Weiterhin ist ein Kohlenwasserstoff-Gemisch bevorzugt, das mindestens 2 voneinander verschiedene Kohlenwasserstoffe enthält, deren Kohlenstoff Zahl sich um mehr als 1 unterscheidet, wobei diese 2 voneinander verschiedene Kohlenwasserstoffe mindestens 60 Gew.-%, vorzugsweise mindestens 70 Gew.-% - bezogen auf die Summe der Kohlenwasserstoffe ausmachen.

Der Begriff "2 voneinander verschiedene Kohlenwasserstoffe" bezeichnet Kohlenwasserstoffe mit einer unterschiedlichen C-Zahl.

Das bedeutet, wenn das Kohlenwasserstoff Gemisch einen Kohlenwasserstoff mit einer C Zahl von n (n = ganzzahlige Zahl) enthält, so enthält das Gemisch mindestens noch einen weiteren Kohlenwasserstoff mit einer C-Zahl von größer gleich n+2 bzw. kleiner gleich n-2.

Vorzugsweise ist n eine ungrade Zahl, insbesondere 7,9,11,13,15,17,19, 21 und/oder 23.

Weiterhin kann als Kohlenwasserstoff ein Kohlenwasserstoff Gemisch eingesetzt werden, das $^{14}$C- Isotope enthält und wobei das Kohlenwasserstoff Gemisch mindestens 2 voneinander verschiedene Kohlenwasserstoffe enthält, deren C-Zahl sich um mehr als 1 unterscheidet.

Ein weiterer wesentlicher Bestandteil der Mikroemulsionen ist Wasser. Das Wasser sollte vorzugsweise demineralisiert sein. Die Mikroemulsionen enthalten vorzugsweise bis zu 81 Gew.-% Wasser. Bevorzugte Bereiche sind Mengen von 30 bis 80 Gew.-% und insbesondere von 45 bis 65 Gew.-% Wasser.

Neben den oben beschriebene Inhaltsstoffen können die Mikroemulsionen als zusätzlichen Bestandteil noch Fettalkohole der allgemeinen Formel R$^2$-OH enthalten, wobei R$^2$ für einen gesättigten oder ungesättigten, verzweigten oder unverzweigten Alkyl- oder Alkenylrest mit 6 bis 22 C-Atomen steht, enthalten kann. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol. Besonders bevorzugt ist die Mitverwendung von Cetylalkohol, Stearylalkohol Arachylalkohol und Behenylalkohol sowie deren Mischungen. Wenn Fettalkohole enthalten sind werden sie bevorzugt in Mengen bis zu 15 Gew.-%, bezogen auf die gesamte Mikroemulsion eingesetzt, wobei der Bereich von 1 bis 10 Gew.-% und vorzugsweise 2 bis 8 Gew.-% besonders bevorzugt sein können. Auch diese Fettalkohole, die wasserunlösliche organische Bestandteile darstellen fallen erfindungsgemäß nicht unter die Definition des Ölkörpers.

Die Mikroemulsion, die im ersten Schritt des erfindungsgemäßen Verfahrens hergestellt wird, kann weiterhin noch anionische Tenside enthalten. Typische Beispiele für anionische Tenside sind Seifen, Alkylbenzolsulfonate, Alkansulfonate, Olefinsulfonate, α-Methylestersulfonate, Sulfofettsäuren, Alkylsulfate, Mono- und Dialkylsulfosuccinate, Mono- und Dialkylsulfosuccinamate, Sulfotriglyceride, Monoglyceridsulfate, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate und Proteinfettsäurekondensate (insbesondere pflanzliche Produkte auf Weizenbasis).

Die im erfindungsgemäßen Verfahren eingesetzten Mikroemulsionen können noch weitere nichtionische, amphotere und/oder kationische Tenside, vorzugsweise in Mengen von insgesamt 1 bis 25 Gew.-%, bezogen auf das Gesamtgewicht der Emulsion, enthalten. Typische Beispiele für weitere nichtionische Tenside (neben dem Alkyl(oligo)glycosiden) sind z.B. Fettsäure-N-alkylglucamide, Polyolfettsäureester, Zuckerester, Sorbitanester, Polysorbate, Alkoholethoxylate und Aminoxide.

Beispiele für geeignete amphotere bzw. zwitterionische Tenside sind Alkylbetaine, Alkylamidobetaine, Aminopropionate, Aminoglycinate, Imidazoliniumbetaine und Sulfobetaine. Beispiele für geeignete Alkylbetaine stellen die Carboxyalkylierungsprodukte von sekundären und insbesondere tertiären Aminen dar. Typische Beispiele sind die Carboxymethylierungsprodukte von Hexylmethylamin, Hexyldimethylamin, Octyldimethylamin, Decyldimethylamin, Dode-

4

cylmethylamin, Dodecyldimethylamin, Dodecylethylmethylamin, $C_{12/14}$-Kokosalkyldimethylamin, Myristyldimethylamin, Cetyldimethylamin, Stearyldimethylamin, Stearylethylmethylamin, Oleyldimethylamin, $C_{16/18}$-Talgalkyldimethylamin sowie deren technische Gemische. Weiterhin kommen auch Carboxyalkylierungsprodukte von Amidoaminen in Betracht. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von $C_{8/18}$-Kokosfettsäure-N,N-dime-thylaminopropylamid mit Natriumchloracetat. Weiterhin kommen auch Imidazoliniumbetaine in Betracht. Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum $C_{12/14}$-Kokosfettsäure, die anschließend mit Natriumchloracetat betainisiert werden.

Typische Beispiele für kationische Tenside sind quartäre Ammoniumverbindungen und Esterquats, insbesondere quaternierte Fettsäuretrialkanolaminestersalze.

Die Mikroemulsionen sind wie folgt zusammengesetzt:

| | |
|---|---|
| Alkyl(oligo)glycoside | 10 bis 20 Gew.-% |
| Monoester des Glycerins von C12-C22 Fettsäuren | 4 bis 10 Gew.-% |
| Ölkörper | 5 bis 30 Gew.-% |

Der Rest auf 100 Gew.-% ist dann jeweils Wasser, ggf. ergänzt um weitere, optionale Inhaltsstoffe.

Kationische Polymere

Als Komponente (c) enthalten die kosmetischen Mittel der vorliegenden Patentanmeldung kationische Polymere. Diese sind bevorzugt gewählt aus der Gruppe der Homo- oder Copolymere von Ester- oder Amidderivaten der Acryl- oder Methacrylsäure (z.B. INCI: Polyquaternium-7), Homopolymeren aus Methacryloylethyltrimethylammonium Chlorid (INCI: Polyquaternium-37), quaternären Copolymeren aus Hydroxyethylcellulose und Diallyldimethyl-ammoniumchlorid (INCI: Polyquaternium-4), polymeren quaternisierten Ammoniumsalzen von Hydroxyethylcellulose, welche mit einem trimethylammoniumsubstituierten Epoxid modifiziert sind (INCI: Polyquaternium-10, Polyquaternium-67), depolymerisierten Guar Gum Derivaten, welche quaternisiert sind (INCI: Guar Hydroxypropyl Trimonium Chlorid), amphotere Copopolymere (INCI: Polyquaternium-74) oder quaternisierte Guarderivate und quaternären Copolymeren aus Hydroxyethylcellulose und Diallyl-demethylammoniumchlorid. In einer bevorzugten Ausführungsform wird das kationische Polymer (c) ausgewählt aus der Gruppe, die gebildet wird von Polyquaternium-7, Polyquaternium-10 und kationischen Guar-Derivaten. In einer besonders bevorzugten Ausführungsform werden kationische Guar-Derivate bevorzugt, da diese dem "grünen" Konzept entsprechen. Die erfindungsgemäßen Zubereitungen enthalten vorzugsweise 0,05 bis 2 Gew.% dieser kationischen Polymere.

Bevorzugt ist eine kosmetische Zubereitung enthaltend

(a) Alkylsulfate und Cocamidopropylbetain,
(b) eine Mikroemulsion enthaltend
(b1) 10 bis 20 Gew.% Alkyloligoglycoside,
(b2) 4 bis 10 Gew.% Monoester von Glycerin mit einer gesättigten oder ungsättigten Fettsäure der Kettenlänge C12-22 und
(b3) 5-30 Gew.% Ölkörper und
(b4) Wasser, und
(c) ein kationisches Polymer ausgewählt aus der Gruppe, die gebildet wird von Polyquaternium-7, Polyquaternium-10 und kationischen Guar-Derivaten.

Besonders bevorzugt ist eine kosmetische Zubereitung enthaltend

(a) 5 - 20 Gew.% bezogen auf die Gesamtzusammensetzung Alkylsulfate und Cocamidopropylbetain,
(b) 0,5 - 10 Gew.% bezogen auf die Gesamtzusammensetzung einer Mikroemulsion enthaltend (Mengenangaben für die Komponenten (b1) bis (b4) bezogen auf die Mikroemulsion)

(b1) 10 - 20 Gew.% Alkyloligoglycoside,

(b2) 4 - 10 Gew.% Monoester von Glycerin mit einer gesättigten oder ungesättigten Fettsäure der Kettenlänge C 12-22,

(b3) 5 - 30 Gew.% eines Ölkörpers und

(b4) Rest auf 100 Gew.% Wasser, und

(c) 0,05 - 1 Gew.% bezogen auf die Gesamtzusammensetzung eines kationischen Polymers ausgewählt aus der Gruppe, die gebildet wird von Polyquaterinium-7, Polyquaternium-10 und kationischen Guar-Derivaten.

Ein weiterer Gegenstand der vorliegenden Erfindung ist ein Verfahren zur Herstellung einer kosmetischen Zubereitung gemäß Anspruch 5.

**Beispiele**

Es wurde zunächst eine Mikroemulsion der folgenden Zusammensetzung durch Vermischen der Inhaltsstoffe hergestellt (Tab. 1 und 2):

Tabelle 1: Zusammensetzung einer Mikromemulsion gemäß Komponente b) der Erfindung:

| Substanz | INCI | Gew.-% Aktivsubstanz |
|---|---|---|
| Plantacare® 2000 UP | Decyl Glucoside | 17,5 |
| Monomuls® 90 O 18 | Glyceryl Oleate | 8 |
| Cetiol® OE | Dicaprylyl Ether | 20 |
| Aqua dem. | | add 100 |

Tabelle 2: Shampoo-Formulierungen und Leistung bei der Nasskämmarbeit

| Substanz | INCI | Gew.% Aktivsubstanz | | | |
|---|---|---|---|---|---|
| Sulfopon® 1216G | Sodium C12-16 fatty alcohol sulfate | 4,8 | 4,8 | - | - |
| Texapon® ALS Benz | Ammonium Lauryl Sulfate | - | - | 5,3 | 5,3 |
| Plantacare® 2000 UP | Decyl Glucoside | 8,1 | 8,1 | - | - |
| Plantapon® LGC Sorb | Sodium Lauryl Glucose Carboxylate (and) Lauryl Glucoside | - | - | 2,7 | 2,7 |
| Dehyton® PK 45 | Cocamidopropyl Betaine | 2,1 | 2,1 | 3,1 | 3,1 |
| Emulsion gem. Tab. 1 | | - | 7,0 | - | 18,0 |
| Jaguar® C 162 | Hydroxypropyl Guar Hydroxypropyl trimonium Chloride | 0,2 | 0,2 | 0,2 | 0,2 |
| Keltrol® CG-SFT | Xanthan Gum | 1,0 | 1,0 | 1,0 | 1,0 |
| Sodium Benzoate | Sodium Benzoate | 0,5 | 0,5 | 0,5 | 0,5 |
| Citric Acid 50% (pH 5) | Citric Acid | q.s. | q.s. | q.s. | q.s. |
| Aqua dem. | | ad 100 | | | |
| Restkämmarbeit [%] | | 100 | 56 | 100 | 39 |

Die Untersuchungen hinsichtlich der Konditionierleistung der Shampoos wurden jeweils an 10 Haarsträhnen in einem automatisierten System zur Bestimmung der Nasskämmarbeit durchgeführt.

Die Vorbehandlung der Haarsträhnen (12cm/1g) der Firma IHIP wurden in einem automatisierten Haarbehandlungssystem durchgeführt:

- 30 min Reinigung mit 6% Natriumlaurylethersulfat, pH 6.5, danach intensives Spülen der Haare
- 20 min Bleiche mit einer Lösung von 5 % Wasserstoffperoxid, pH 9.4 (mit Ammoniumhydroxidlösung eingestellt), danach intensives Spülen der Haare

- 30 min Trocknung in einem Luftstrom bei 68°C

Direkt vor der Nullmessung wurden die Haare für 30 Minuten in Wasser gequollen und anschließend mit einer automatischen Nassauskämmapparatur für 1 Minute ausgespült. Im automatisierten System zur Bestimmung der Nass- und Trockenkämmarbeit wurden die Kämmkräfte während 20 Kämmungen bestimmt und die Kämmarbeit durch Integration der gemessenen Kraft-Weg-Kurven errechnet. Nach der Nullmessung wurden die Haare sofort mit der Formulierung behandelt (0,25g/g Haar). Nach 5 Minuten Einwirkzeit wurde mit der automatischen Nassauskämmapparatur unter Standardbedingungen (38°C, 1l/Minute) gespült.

Die Behandlung und das folgende Ausspülen wurde ein zweites Mal wiederholt. Dann erfolgte die Vergleichsmessung (zur Nullmessung). Die Messungen wurden mit der feinen Kammseite der Naturkautschukkämme durchgeführt. Berechnet wurde die Restkämmarbeit pro Strähne wie folgt:

$$\text{Restkämmarbeit} = \text{Kämmarbeit nach Produktbehandlung} / \text{Kämmarbeit vor Produktbehandlung}$$

Anschließend wurde über die Quotienten aller 10 Strähnen der Mittelwert und die Standardabweichung bestimmt.

## Patentansprüche

1.  Kosmetische Zubereitung enthaltend

    (a) mindestens ein Tensid ausgewählt aus nicht-alkoxylierten anionischen, zwitterionischen oder amphoteren Tensiden,
    (b) eine Mikroemulsion mit einer mittleren Teilchengröße von weniger als 1 $\mu$m enthaltend
    10 bis 20 Gew.% Alkylpolyglykoside
    4 bis 10 Gew.% Monoester des Glycerins von C12-C22 Fettsäuren
    5 bis 30 Gew.% Ölkörper und
    (c) mindestens ein kationisches Polymer.

2.  Zubereitung gemäß Anspruch 1, **dadurch gekennzeichnet, dass** das kationische Polymer (c) ausgewählt ist aus der Gruppe, die gebildet wird von Polyquaternium-7, Polyquaternium-10 und kationischen Guar-Derivaten.

3.  Zubereitung gemäß Anspruch 1, **dadurch gekennzeichnet, dass** das kationische Polymer ein kationisches Guar-Derivat ist.

4.  Zubereitung gemäß einem der Ansprüche 1 bis 3, **dadurch gekennzeichnet, dass** als Komponente (a) ein Alkylsulfat oder eine Mischung von Alkylsulfat und Cocamidopropylbetain eingesetzt wird.

5.  Verfahren zur Herstellung einer kosmetischen Zubereitung, indem

    (a) mindestens ein Tensid ausgewählt aus nicht-alkoxylierten anionischen, zwitterionischen oder amphoteren Tensiden,
    (b) eine Mikroemulsion mit einer mittleren Teilchengröße von weniger als 1 $\mu$m enthaltend
    10 bis 20 Gew.% Alkylpolyglykoside
    4 bis 10 Gew.% Monoester des Glycerins von C12-C22 Fettsäuren
    5 bis 30 Gew.% Ölkörper und
    c) mindestens ein kationisches Polymer

    mit weiteren kosmetischen Grundstoffen verrührt.

## Claims

1.  A cosmetic preparation comprising

(a) at least one surfactant selected from non-alkoxylated anionic, zwitterionic or amphoteric surfactants,
(b) a microemulsion having an average particle size of less than 1 $\mu$m, comprising
10 to 20% by weight of alkyl polyglycosides
4 to 10% by weight of monoesters of glycerol of C12-C22 fatty acids
5 to 30% by weight of oil bodies and
(c) at least one cationic polymer.

2. The preparation according to claim 1, wherein the cationic polymer (c) is selected from the group which is formed by polyquaternium-7, polyquaternium-10 and cationic guar derivatives.

3. The preparation according to claim 1, wherein the cationic polymer is a cationic guar derivative.

4. The preparation according to any one of claims 1 to 3, wherein an alkyl sulfate or a mixture of alkyl sulfate and cocamidopropylbetaine is used as component (a).

5. A process for preparing a cosmetic preparation by stirring

(a) at least one surfactant selected from non-alkoxylated anionic, zwitterionic or amphoteric surfactants,
(b) a microemulsion having an average particle size of less than 1 $\mu$m, comprising
10 to 20% by weight of alkyl polyglycosides
4 to 10% by weight of monoesters of glycerol of C12-C22 fatty acids
5 to 30% by weight of oil bodies and
(c) at least one cationic polymer

with further cosmetic base substances.

**Revendications**

1. Préparation cosmétique contenant :

(a) au moins un tensioactif choisi parmi les tensioactifs non alcoxylés anioniques, zwitterioniques ou amphotères,
(b) une microémulsion ayant une taille de particule moyenne de moins de 1 $\mu$m, contenant
10 à 20 % en poids de polyglycosides d'alkyle,
4 à 10 % en poids de monoesters de glycérine d'acides gras en C12-C22,
5 à 30 % en poids de corps huileux et
(c) au moins un polymère cationique.

2. Préparation selon la revendication 1, **caractérisée en ce que** le polymère cationique (c) est choisi dans le groupe formé par le polyquaternium-7, le polyquaternium-10 et les dérivés de guar cationiques.

3. Préparation selon la revendication 1, **caractérisée en ce que** le polymère cationique est un dérivé de guar cationique.

4. Préparation selon l'une quelconque des revendications 1 à 3, **caractérisée en ce qu'**un sulfate d'alkyle ou un mélange d'un sulfate d'alkyle et de cocamidopropylbétaïne est utilisé en tant que composant (a).

5. Procédé de fabrication d'une préparation cosmétique, selon lequel

(a) au moins un tensioactif choisi parmi les tensioactifs non alcoxylés anioniques, zwitterioniques ou amphotères,
(b) une microémulsion ayant une taille de particule moyenne de moins de 1 $\mu$m, contenant
10 à 20 % en poids de polyglycosides d'alkyle,
4 à 10 % en poids de monoesters de glycérine d'acides gras en C12-C22,
5 à 30 % en poids de corps huileux et
(c) au moins un polymère cationique,

sont mélangés avec d'autres matières de base cosmétiques.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004030885